# EUROPEAN PATENT APPLICATION

(11) **EP 1 629 841 A1**
(43) Date of publication of application: **01.03.2006**
(21) Application number: 04735386.7
(22) Date of filing: 28.05.2004
(51) Int. Cl.: A61K 31/404, A61K 31/7056, A61P 25/24, A23L 1/30, C07D 405/04, C07H 7/06

(54) **ANTIDEPRESSANTS OR FOOD AND DRINKS FOR ANTIDEPRESSION**

(30) Priority: 29.05.2003 JP 2003153132
(71) Applicant: KYOWA MEDEX CO., LTD., Chuo-ku, Tokyo 104-6004 (JP); KYOWA HAKKO KOGYO CO., LTD., Chiyoda-ku, Tokyo 100-8185 (JP)
(72) Inventor: KOHNO, Hiroaki c/o Kyowo Medex Research Lab, Kyowa, Nagaizumo-cho,Sunto-gun,Shizuoka 4110932 (JP); KUSAKA, Hideaki c/o Pharmaceutical Research Center, Nagaizumi-cho,Sunto-gun,Shizuoka,4118731 (JP)
(74) Representative: Harding, Charles Thomas
(86) International application number: PCT/JP2004/007768
(87) International publication number: WO 2004/105753

(57) **Abstract**

An object of the present invention is to provide antidepressants or foods and drinks for antidepression. To solve the present problem, the present invention provides antidepressants or foods and drinks for antidepression comprising, as an active ingredient, a compound represented by formula (I): 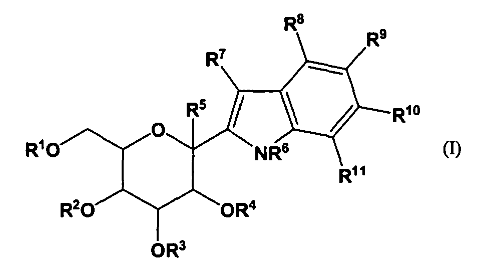 (wherein R¹, R², R³ and R⁴, which may be the same or different, each represent a hydrogen atom, substituted or unsubstituted lower alkyl, or the like.; R⁵ represents a hydrogen atom, hydroxy, or the like.; R⁶ represents a hydrogen atom, or the like.; R⁷ represents a hydrogen atom or substituted or unsubstituted lower alkyl; and R⁸, R⁹, R¹⁰ and R¹¹, which may be the same or different, each represent a hydrogen atom or substituted or unsubstituted lower alkyl) or a salt thereof.

## Description

### Technical Field

The present invention relates to antidepressants or foods and drinks for antidepression.

### Background Art

As the antidepressants, monoamine oxidase (MAO) inhibitors, tricyclic antidepressants such as amitriptyline and imipramine, and tetracyclic antidepressants such as mianserin and maprotiline have been conventionally used. However, they are problematic in that MAO inhibitors have side effects such as hepatopathy, excessive stimulant action, toxic psychosis, sleeplessness, orthostatic hypotension, vertigo, ear noises and constipation, tricyclic antidepressants have side effects such as drowsiness, systemic lassitude, anticholinergic effects (e.g., dry mouth, constipation, urinary disturbance, tachycardia and aggravation of glaucoma), orthostatic hypotension, hepatopathy, efflorescence, delirium, vertigo and spasm, and tetracyclic antidepressants have side effects such as spasm, drug eruption, drowsiness and granulocytopenia. On the other hand, serotonin (5-HT) has been reported to be related to antidepressive activity [The Brain Receptor (New Version), Norio Ogawa, ed., Sekai Hoken Tsushinsha (1991), etc.]. The correlation between 5-HT reuptake inhibition or 5-HT receptor and antidepressive activity has been studied, and selective serotonin reuptake inhibitors (hereinafter referred to as SSRI) such as fluoxetine, sertraline and paroxetine have been developed as antidepressants. However, even these SSRIs are known to have side effects such as hypotension, palpitation, vertigo, dizziness, drowsiness, efflorescence, hepatopathy, lassitude, vomiting and nausea.

On the other hand, a method of examining vital functions using 2-amino-3-[2-(α-mannopyranosyl)indol-3-yl]propionic acid derivatives is reported (WO99/09411). A psychotropic comprising a 2-amino-3-[2-(*α-*mannopyranosyl)indol-3-yl]propionic acid derivative is also reported (Japanese Patent No. 3394280).

### Disclosure of the Invention

An object of the present invention is to provide antidepressants or foods and drinks for antidepression.

The present invention relates to the following (1) to (19).
(1) An antidepressant comprising, as an active ingredient, a compound represented by formula (I): 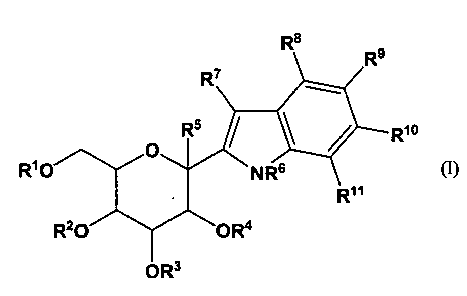 (wherein R¹, R², R³ and R⁴, which may be the same or different, each represent a hydrogen atom, substituted or unsubstituted lower alkyl, substituted or unsubstituted alkenyl, or substituted or unsubstituted aryl; R⁵ represents a hydrogen atom, hydroxy, or substituted or unsubstituted lower alkoxy; R⁶ represents a hydrogen atom, substituted or unsubstituted lower alkyl, substituted or unsubstituted sulfamoyl, substituted or unsubstituted arylsulfonyl, substituted or unsubstituted lower alkoxycarbonyl, or substituted or unsubstituted aralkyloxycarbonyl; R⁷ represents a hydrogen atom or substituted or unsubstituted lower alkyl; and R⁸, R⁹, R¹⁰ and R¹¹, which may be the same or different, each represent a hydrogen atom or substituted or unsubstituted lower alkyl) or a salt thereof.
(2) A food and drink for antidepression comprising, as an active ingredient, a compound represented by formula (I) : 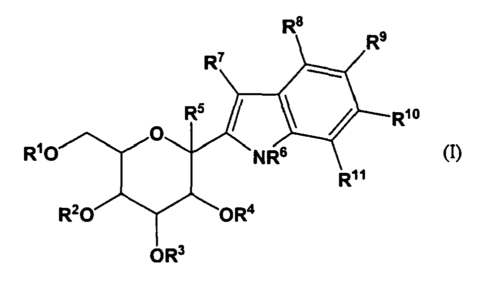 (wherein R¹, R², R³, R⁴, R⁵, R⁶, R⁷, R⁸, R⁹, R¹⁰ and R¹¹ each have the same meaning as defined above) or a salt thereof.
(3) The antidepressant according to the above (1), wherein R⁵ is a hydrogen atom or hydroxy; and R⁸, R⁹, R¹⁰ and R¹¹ each are a hydrogen atom.
(4) The antidepressant according to the above (1), wherein R⁷, R⁸, R⁹, R¹⁰ and R¹¹ each are a hydrogen atom.
(5) The food and drink for antidepression according to the above (2), wherein R⁵ is a hydrogen atom or hydroxy; and R⁸, R⁹, R¹⁰ and R¹¹ each are a hydrogen atom.
(6) The food and drink for antidepression according to the above (2), wherein R⁷, R⁸, R⁹, R¹⁰ and R¹¹ each are a hydrogen atom.
(7) An antidepressant comprising, as an active ingredient, a compound represented by formula (Ia) or (Ib): 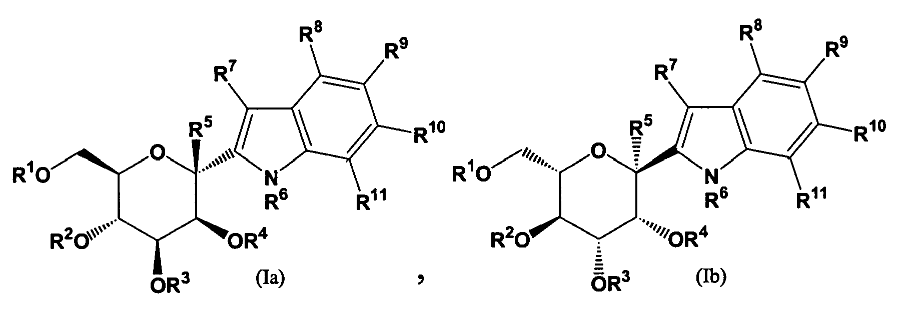 (wherein R¹, R², R³, R⁴, R⁵, R⁶, R⁷, R⁸, R⁹, R¹⁰ and R¹¹ each have the same meaning as defined above) or a salt thereof.
(8) A food and drink for antidepression comprising, as an active ingredient, a compound represented by formula (Ia) or (Ib): 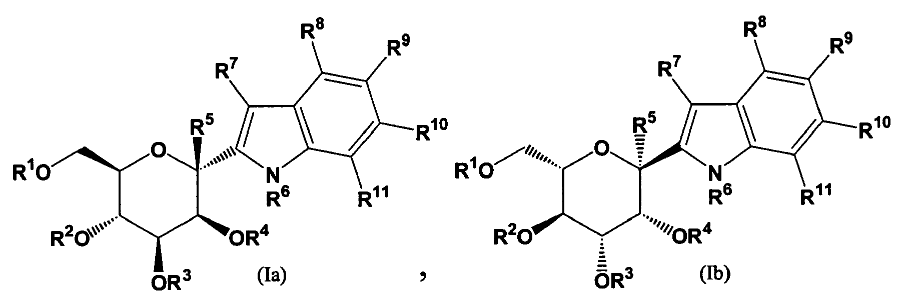 (wherein R¹, R², R³, R⁴, R⁵, R⁶, R⁷, R⁸, R⁹, R¹⁰ and R¹¹ each have the same meaning as defined above) or a salt thereof.
(9) An antidepressant comprising, as an active ingredient, 2-amino-3-[2-(α-D-mannopyranosyl)indol-3-yl]propionic acid represented by formula (II): 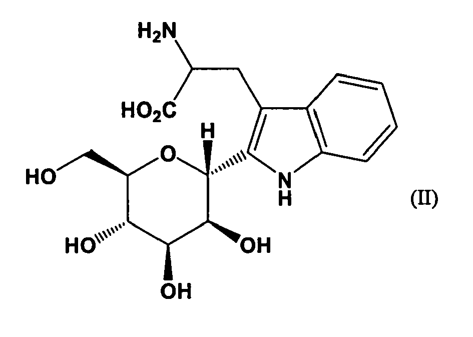 or a salt thereof.
(10)An antidepressant comprising, as an active ingredient, 2-amino-3-[2-(*α*-L-mannopyranosyl)indol-3-yl]propionic acid represented by formula (III): 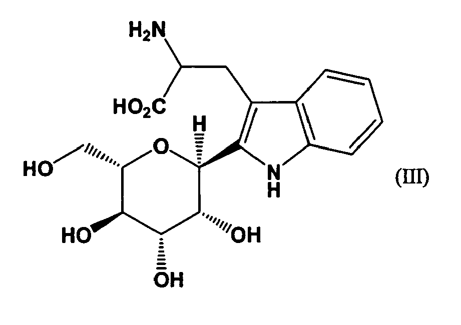 or a salt thereof.
(11) A food and drink for antidepression comprising, as an active ingredient, 2-amino-3-[2-(*α*-D-mannopyranosyl)indol-3-yl]propionic acid represented by formula (II): 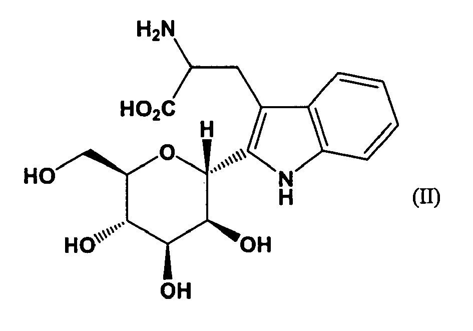 or a salt thereof.
(12) A food and drink for antidepression comprising, as an active ingredient, 2-amino-3-[2-(*α*-L-mannopyranosyl)indol-3-yl]propionic acid represented by formula (III): 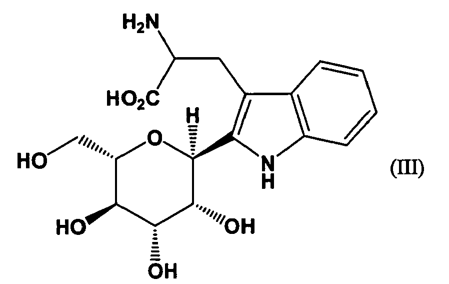 or a salt thereof.
(13)An antidepressant comprising, as an active ingredient, a compound represented by formula (IV): 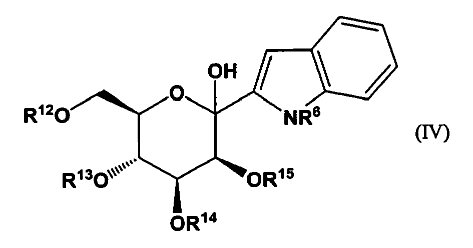 (wherein R⁶ has the same meaning as defined above; and R¹², R¹³, R¹⁴ and R¹⁵, which may be the same or different, each represent substituted or unsubstituted lower alkyl, substituted or unsubstituted alkenyl, or substituted or unsubstituted aryl) or a salt thereof.
(14)An antidepressant comprising, as an active ingredient, a compound represented by formula (V): 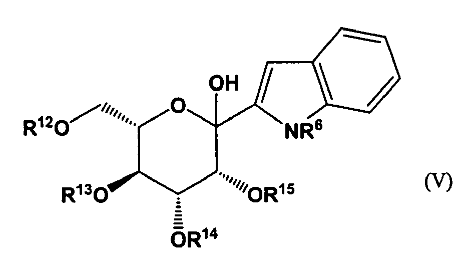 (wherein R⁶, R¹², R¹³, R¹⁴ and R¹⁵ each have the same meaning as defined above) or a salt thereof.
(15) A food and drink for antidepression comprising, as an active ingredient, a compound represented by formula (IV) : 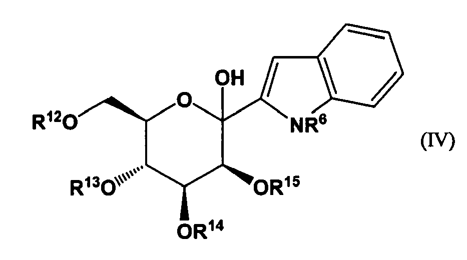 (wherein R⁶, R¹², R¹³, R¹⁴ and R¹⁵ each have the same meaning as defined above) or a salt thereof.
(16) A food and drink for antidepression comprising, as an active ingredient, a compound represented by formula (V) : 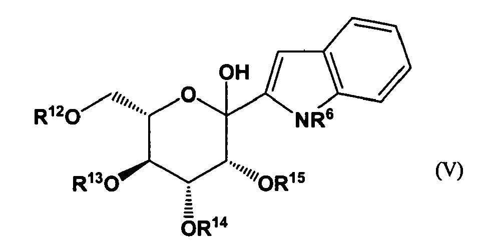 (wherein R⁶, R¹², R¹³, R¹⁴ and R¹⁵ each have the same meaning as defined above) or a salt thereof.
(17) A method of preventing or treating depression which comprises administering an effective amount of a compound represented by formula (I) according to the above (1), formula (Ia) or (Ib) according to the above (7), formula (II) according to the above (9), formula (III) according to the above (10), formula (IV) according to the above (13) or formula (V) according to the above (14) or a salt thereof.
(18) Use of a compound represented by formula (I), (Ia), (Ib), (II), (III), (IV) or (V) or a salt thereof for the manufacture of the antidepressant according to any of the above (1), (3), (4), (7), (9), (10), (13) and (14).
(19) Use of a compound represented by formula (I), (Ia), (Ib), (II), (III), (IV) or (V) or a salt thereof for the manufacture of the food and drink for antidepression according to any of the above (2), (5), (6), (8), (11), (12), (15) and (16).

Hereinafter, the compounds represented by formulae (I), (Ia), (Ib), (II), (III), (IV) and (V) are referred to as Compounds (I), (Ia), (Ib), (II), (III), (IV) and (V), respectively.

The definitions of the groups in formulae (I), (Ia), (Ib), (IV) and (V) are explained below.

The lower alkyl and the lower alkyl moiety of the lower alkoxy and the lower alkoxycarbonyl include straight-chain or branched alkyl groups having 1 to 6 carbon atoms, such as methyl, ethyl, propyl, isopropyl, butyl, isobutyl, sec-butyl, tert-butyl, pentyl, isopentyl, neopentyl and hexyl.

The alkenyl includes straight-chain or branched alkenyl groups having 2 to 6 carbon atoms, such as vinyl, allyl, 1-propenyl, methacryl, crotyl, 1-butenyl, 3-butenyl, 2-pentenyl, 4-pentenyl, 2-hexenyl and 5-hexenyl.

The aryl and the aryl moiety of the arylsulfonyl and the aralkyloxylcarbonyl include aryl groups having 6 to 14 carbon atoms, such as phenyl, naphthyl and anthryl.

The alkylene moiety of the aralkyloxycarbonyl has the same meaning as the above-described lower alkyl except one hydrogen atom is removed therefrom.

The substituted lower alkyl, the substituted lower alkoxy, the substituted lower alkoxycarbonyl and the substituted alkenyl each have 1 to 3 substituents which are the same or different. Examples of the substituents are halogen, amino, nitro, hydroxy, carboxy, carbamoyl, sulfo, tri-lower alkylsilyl, lower alkoxy, aryl, substituted or unsubstituted mono- or di-lower alkylamino, substituted or unsubstituted mono or bis(aralkyloxycarbonyl)amino and substituted or unsubstituted lower alkoxycarbonyl. Herein, the halogen means fluorine, chlorine, bromine and iodine atoms; the lower alkyl moiety of the lower alkoxy, the tri-lower alkylsilyl, the mono- or di-lower alkylamino and the lower alkoxycarbonyl has the same meaning as the above-described lower alkyl; the three lower alkyl moieties of the tri-lower alkylsilyl and the two lower alkyl moieties of the di-lower alkylamino each may be the same or different; the aryl and the aryl moiety of the mono or bis(aralkyloxycarbonyl)amino have the same meaning as the above-described aryl; the alkylene moiety of the mono or bis(aralkyloxycarbonyl)amino has the same meaning as the above-described lower alkyl except one hydrogen atom is removed therefrom; and the two aralkyloxycarbonyl moieties of the bis(aralkyloxycarbonyl)amino may be the same or different. The substituted mono- or di-lower alkylamino, the substituted mono or bis(aralkyloxycarbonyl)amino and the substituted lower alkoxycarbonyl each have 1 to 3 substituents which are the same or different. Examples of the substituents are halogen, amino, nitro, hydroxy, carboxy, carbamoyl and sulfo. Herein, the halogen has the same meaning as defined above.

The substituted aryl, the substituted arylsulfonyl and the substituted aralkyloxycarbonyl each have 1 to 3 substituents which are the same or different. Examples of the substituents are halogen, amino, nitro, hydroxy, carboxy, carbamoyl, sulfo, lower alkyl and lower alkoxy. Herein, the halogen has the same meaning as defined above, and the lower alkyl and the lower alkyl moiety of the lower alkoxy have the same meaning as the above-described lower alkyl.

The substituted sulfamoyl has 1 or 2 substituents which are the same or different. Examples of the substituents are substituted or unsubstituted lower alkyl and substituted or unsubstituted aryl. Herein, the lower alkyl and the aryl each have the same meaning as defined above, and the substituent in the substituted lower alkyl and the substituent in the substituted aryl each have the same meaning as defined above.

The salts of Compound (I), (Ia), (Ib), (II), (III), (IV) or (V) are preferably pharmaceutically acceptable ones, such as acid addition salts (e.g., acetate, hydrochloride, sulfate, nitrate, citrate, methanesulfonate, maleate and fumarate), alkali metal salts (e.g., sodium salt and potassium salt), alkaline earth metal salts (e.g., magnesium salt and calcium salt), metal salts (e.g., aluminum salt and zinc salt), ammonium salts (e.g., ammonium salt and tetramethylammonium salt) and organic amine addition salts (e.g., triethylamine salt, morpholine salt and piperazine salt).

Compounds (I), (Ia), (Ib), (II), (III), (IV) and (V) and salts thereof can be obtained according to, for example, the method described in WO99/09411, etc.

The pharmacological activities of Compound (I), (Ia), (Ib), (II), (III), (IV) or (V) are illustrated in detail in test examples. As the test compound, 2-amino-3-[2-(*α-*L-mannopyranosyl]indol-3-yl]propionic acid was used. This compound is referred to as Compound 1 in the present specification.

### Test Example 1

### Action on Reserpine-induced Depressive Behavior

### 1) Action on Body Temperature

Male ddY mice (4 weeks old) were subcutaneously administered reserpine (2 mg/kg) (Apoplon injection 1 mg, product of Daiichi Pharmaceutical Co., Ltd.). These mice were divided into two groups: one to which Compound 1 (100 mg/kg) was intraperitoneally administered once per day for two days (reserpine + Compound 1-administered group) and the other to which only a solvent was administered (reserpine + solvent-administered group). Reserpine and Compound 1 were administered after being dissolved in a physiological saline. The body temperature of the mice was measured 0.5, 1, 3 and 6 hours after the last administration.

Separately, a solvent was administered to another group of mice without reserpine administration. The results are shown in Table 1. A significant temperature drop by the administration of reserpine was observed, while Compound 1 significantly raised the body temperature of the reserpine-administered mice.

**Table 1 Action of Compound 1 on reserpine-induced temperature drop**

| Group | Compound 1 (mg/kg, i.p.) | n | Body temperature (°C) | | | |
|---|---|---|---|---|---|---|
| | | | Time after administration (minute) | | | |
| | | | 30 | 60 | 180 | 360 |
| Solvent | | | *** | *** | *** | *** |
| (s.c.) + solvent | - | 7 | 36.9 ± 0.1 | 36.9 ± 0.1 | 36.7 ± 0.1 | 36.7 ± 0.1 |
| Reserpine | | | | | | |
| (s.c.) + solvent | - | 7 | 33.9 ± 0.2 | 33.5 ± 0.4 | 32.6 ± 0.6 | 33.3 ± 0.3 |
| Reserpine | | | *** | ** | ** | ** |
| (s.c.) + Compound 1 | 100 | 7 | 36.1 ± 0.2 | 35.4 ± 0.1 | 35.2 ± 0.3 | 34.9 ± 0.4 |

| | | | | | | |
|---|---|---|---|---|---|---|
| Data are shown as mean ± standard error. | | | | | | |
| ***: p<0.001, **: p<0.01 vs reserpine (s.c.) + solvent-administered group | | | | | | |
| (Student's t test or Aspin-Welch's t test) | | | | | | |

### 2) Action on Blepharoptosis

Male ddY mice (4 weeks old) were subcutaneously administered reserpine (2 mg/kg) (Apoplon injection 1 mg, product of Daiichi Pharmaceutical Co., Ltd.). These mice were divided into two groups: one to which Compound 1 (100 mg/kg) was intraperitoneally administered once per day for two days (reserpine + Compound 1-administered group) and the other to which only a solvent was administered (reserpine + solvent-administered group). Reserpine and Compound 1 were administered after being dissolved in a physiological saline. The level of blepharoptosis was scored 0.5, 1, 3 and 6 hours after the last administration (score 1: completely opened, score 2: 1/4 closed, score 3: 1/2 closed, score 4: 3/4 closed, and score 5: completely closed). Separately, a solvent was administered to another group of mice without reserpine administration. The results are shown in Table 2. Most of the mice completely closed eyes by the administration of reserpine, while Compound 1 showed the action to improve the condition.

**Table 2 Action of Compound 1 on reserpine-induced blepharoptosis**

| Group | Compound 1 (mg/kg, i.p.) | n | Score | | | |
|---|---|---|---|---|---|---|
| | | | Time after administration (minute) | | | |
| | | | 30 | 60 | 180 | 360 |
| Solvent | | | *** | *** | *** | *** |
| (s.c.) + solvent | - | 7 | 1.0 ± 0.0 | 1.0 ± 0.0 | 1.0 ± 0.0 | 1.0 ± 0.0 |
| Reserpine | | | | | | |
| (s.c.) + solvent | - | 7 | 5.0 ± 0.0 | 5.0 ± 0.0 | 4.7 ± 0.2 | 4.9 ± 0.1 |
| Reserpine | | | *** | ** | ** | ** |
| (s.c.) + Compound 1 | 100 | 7 | 3.0 ± 0.4 | 3.1 ± 0.3 | 3.3 ± 0.3 | 3.3 ± 0.2 |

| | | | | | | |
|---|---|---|---|---|---|---|
| Data are shown as mean ± standard error. | | | | | | |
| ***: p<0.001, **: p<0.01 vs reserpine (s.c.) + solvent-administered group (Wilcoxon's test) | | | | | | |

### 3) Action on the Quantity of Spontaneous Motion

Male ddY mice (4 weeks old) were subcutaneously administered reserpine (2 mg/kg) (Apoplon injection 1 mg, product of Daiichi Pharmaceutical Co., Ltd.). These mice were divided into two groups: one to which Compound 1 (100 mg/kg) was intraperitoneally administered once per day for four days (reserpine + Compound 1-administered group) and the other to which only a solvent was administered (reserpine + solvent-administered group). Reserpine and Compound 1 were administered after being dissolved in a physiological saline. After the last administration, the mice were immediately put into breeding cages equipped with an infrared beam sensor (NS-AS01, Neuroscience, Inc.). After the last administration, the quantity of spontaneous motion in each 30-minute period was measured for 360 minutes with an infrared beam sensor device for measuring the quantity of spontaneous motion (AB system, Neuroscience, Inc.) and the measured values were summed up on a specific purpose computer via an interface (B060CT8H06, Neuroscience, Inc). Separately, another group of mice which was administered no reserpine was tested. The results are shown in Table 3. The quantity of spontaneous motion remarkably dropped by the treatment with reserpine, while Compound 1 showed the action to increase the quantity of spontaneous motion.

**Table 3 Action of Compound 1 on reserpine-induced drop in the spontaneous motion level**

| Group | Compound 1 (mg/kg, i.p.) | n | Quantity of spontaneous motion in 360 minutes (count) |
|---|---|---|---|
| Solvent | | | *** |
| (s.c.) + solvent | - | 6 | 2166.2 ± 124.5 |
| Reserpine | | | |
| (s.c.) + solvent | - | 6 | 370.0 ± 30.4 |
| Reserpine | | | * |
| (s.c.) + Compound 1 | 100 | 6 | 2464.2 ± 594.6 |

| | | | |
|---|---|---|---|
| Data are shown as mean ± standard error. | | | |
| ***: p<0.001, *: p<0.05 vs reserpine (s.c.) + solvent-administered group (Aspin-Welch's t test) | | | |

### Test Example 2

### Action on Depressive Behavior Induced by Vitamin B Deficient Food

Male ddY mice (3 weeks old) were fed with a feed deficient in vitamin B1, vitamin B12 and folic acid for 8 days and then divided into two groups: one to which Compound 1 (100 mg/kg) was intraperitoneally administered once per day for 11 days (vitamin deficient food + Compound 1-administered group) and the other to which only a solvent was administered (vitamin deficient food + solvent-administered group). Compound 1 was administered after being dissolved in a physiological saline. Two hours after the last administration, the mice were put in a plastic cylindrical tub (diameter: 18 cm, height: 40 cm, water depth: 15 cm, water temperature: ca. 23°C), followed by measurement of the time of their struggle, swimming and immobility in 10 minutes. Separately, another group of mice fed with a normal food was tested. The results are shown in Table 4. A reduction in the time of struggle and swimming and an increase in the time of immobility due to the administration of a vitamin B deficient food were observed, while Compound 1 improved these actions.

**Table 4 Action of Compound 1 on the swimming patterns of mice fed with a vitamin B deficient food**

| Group | Compound 1 (mg/kg, i.p.) | n | Swimming pattern | | |
|---|---|---|---|---|---|
| | | | Struggle (second) | Swimming (second) | Immobility (second) |
| Normal | | | | *** | *** |
| food + solvent | - | 7 | 6.6 ± 2.8 | 554.3 ± 12.7 | 39.1 ± 12.7 |
| Vitamin B | | | | | |
| deficient food + solvent | - | 6 | 0.0 ± 0.0 | 375.3 ± 28.4 | 224.7 ± 28.4 |
| Vitamin B | | | *** | *** | *** |
| deficient food + Compound 1 | 100 | 5 | 2.4 ± 2.4 | 562.0 ± 14.2 | 35.6 ± 15.4 |

| | | | | | |
|---|---|---|---|---|---|
| Data are shown as mean ± standard error. | | | | | |
| ***: p<0.001 vs vitamin B deficient food + solvent-administered group (Student's t test) | | | | | |

### Test Example 3

### Action on the Amount of Amine in the Brain

Male ddY mice (4 weeks old) were intraperitoneally administered Compound 1 (100 mg/kg) (Compound 1-administered group) and their brains were excised 30 and 60 minutes after the administration to measure the amounts of serotonin (5-HT) and its metabolite 5-hydroxyindole-3-acetic acid (5-HIAA) in the hypothalamus. Compound 1 was administered after being dissolved in a physiological saline. Separately, another group of mice was administered only a solvent (solvent-administered group) and the amounts of 5-HT and 5-HIAA were measured in the same manner. The results are shown in Table 5. Compound 1 increased the contents of 5-HT and 5-HIAA in the hypothalamus of mice.

**Table 5 Action of Compound 1 on the contents of 5-HT and 5-HIAA in the hypothalamus of mice**

| Group | Compound 1 (mg/kg, i.p.) | n | Content (ng/g wet weight) | |
|---|---|---|---|---|
| | | | 5-HT | 5-HIAA |
| Solvent | - | 3 | 563.6 ± 59.7 | 189.7 ± 13.3 |
| | | | * | ** |
| Compound 1 | 100 | 3 | 815.0 ± 39.1 | 374.1 ± 26.7 |

| | | | | |
|---|---|---|---|---|
| Data are shown as mean ± standard error. | | | | |
| ***: p<0.01, *: p<0.05 vs solvent-administered group (Student's t test) | | | | |

The pharmaceutical agent of the present invention comprises, as an active ingredient, a substance selected from the group consisting of Compounds (I), (Ia), (Ib), (II), (III), (IV) and (V) and salts thereof. As the pharmaceutical agent of the present invention, the above substance which is an active ingredient may be administered as such, but it is generally preferred to administer the pharmaceutical agent in the form of a pharmaceutical composition comprising the above substance as an active ingredient and one or more kinds of additives for pharmaceutical preparation. Such pharmaceutical compositions can be produced according to the method which itself is known or conventional in the field of pharmaceutics. The pharmaceutical agent of the present invention in the form of a pharmaceutical composition may comprise one or more active ingredients of other pharmaceutical agents.

There is no specific restriction as to the administration route of the pharmaceutical agent of the present invention, and the most effective administration route for prevention or treatment can be appropriately selected from oral administration and parenteral administration such as intravenous administration. An example of the preparation suited for oral administration is tablets, and an example of the preparation suited for parenteral administration is an injection.

Solid preparations such as tablets can be produced using, for example, excipients (e.g., lactose and mannitol), disintegrators (e.g., starch), lubricants (e.g., magnesium stearate), binders (e.g., hydroxypropyl cellulose), surfactants (e.g., fatty acid esters) and plasticizers (e.g., glycerin).

Of the preparations suited for parenteral administration, preparations for intravascular administration such as injections can be prepared preferably using an aqueous vehicle which is isotonic to human blood. For example, injections can be prepared as solutions, suspensions or dispersed solutions according to conventional methods using an aqueous vehicle selected from a saline solution, a glucose solution and a mixture of a saline solution and a glucose solution together with appropriate auxiliaries. Preparations for parenteral administration can also be produced using, for example, one or more kinds of additives for pharmaceutical preparation selected from the group consisting of diluents, flavors, antiseptics, excipients, disintegrators, lubricants, binders, surfactants and plasticizers.

There is no specific restriction as to the dose and administration schedule of the pharmaceutical agent of the present invention, and they can be appropriately selected according to various conditions such as the kind of the above substance as an active ingredient, the administration route, the purpose of the treatment or prevention, the age and body weight of a patient, and the nature and degree of severeness of symptom. For example, it is preferred to administer the present pharmaceutical agent in a daily dose of 0.1 to 100 mg/kg per adult in 3 to 4 portions.

However, the dose and administration schedule vary depending upon the various conditions described above.

The food and drink of the present invention can be processed and produced according to general methods for producing foods and drinks, modified only in that a substance selected from the group consisting of Compounds (I), (Ia), (Ib), (II), (III), (IV) and (V) and salts thereof is added to the food and drink.

The food and drink of the present invention can also be produced, for example, by using granulating methods (e.g., fluidized bed granulation, stirring granulation, extrusion granulation, rolling granulation, compression molding granulation, crushing granulation, spray granulation and jet granulation), coating methods (e.g., pan coating, fluidized bed coating and dry coating), puffing methods (e.g., puff drying, excess vapor method, foam-mat method and microwave-heating method), and extrusion methods using an extrusion granulator, an extruder, or the like.

The food and drink of the present invention may be in any of the forms such as a powder food, a sheet-shaped food, a bottled food, a canned food, a retort pouched food, a capsule food, a tablet food, a liquid food and a nutritional drink.

The food and drink of the present invention can be used as a health food or a functional food having antidepressive activity.

The food and drink of the present invention may further comprise food additives generally used in foods and drinks, such as sweeteners, coloring agents, preservatives, thickening stabilizers, antioxidants, color developing agents, bleaching agents, fungicides, gum bases, bitter agents, enzymes, wax, sour agents, seasonings, emulsifiers, nutrient supplements, manufacture facilitating agents, flavors and spice extracts.

The amount of the substance selected from the group consisting of Compounds (I), (Ia), (Ib), (II), (III), (IV) and (V) and salts thereof to be added to the food and drink of the present invention is appropriately determined according to the kind of the food and drink, the effect expected by taking the food and drink, etc., but the substance selected from the group consisting of Compounds (I), (Ia), (Ib), (II), (III), (IV) and (V) and salts thereof is usually added so that its content may become 1 to 100%, preferably 10 to 100%, further preferably 20 to 100%.

The amount of the intake of the food and drink of the present invention varies depending upon the mode of intake, the age and body weight of a taker, etc., but it is preferably 0.1 to 100 mg/kg per adult per day in terms of the substance selected from the group consisting of Compounds (I), (Ia), (Ib), (II), (III), (IV) and (V) and salts thereof, which is administered, i.e. ingested in one to several portions.

### Best Modes for Carrying Out the Invention

Certain embodiments of the present invention are illustrated in the following examples. These examples are not to be construed as limiting the scope of the present invention.

### Example 1 Tablets

Tablets having the following composition are prepared according to a conventional method.

| Formula: | |
|---|---|
| Compound 1 | 20 mg |
| Lactose | 143.4 mg |
| Potato starch | 30 mg |
| Hydroxypropyl cellulose | 6 mg |
| Magnesium stearate | 0.6 mg |
| | 200 mg |

### Example 2 An injection

An injection having the following composition is prepared according to a conventional method.

| Formula: | |
|---|---|
| Compound 1 | 2 mg |
| Purified soybean oil | 200 mg |
| Purified egg yolk lecithin | 24 mg |
| Glycerin for injection | 50 mg |
| Distilled water for injection | 1.72 ml |
| | 2.00 ml |

### Example 3 A refreshing drink

A refreshing drink (10 bottles) having the following composition is prepared according to a conventional method.

| Formula: | |
|---|---|
| Compound 1 | 200 mg |
| Vitamin C | 1 g |
| Vitamin B1 | 5 mg |
| Vitamin B2 | 10 mg |
| Vitamin B6 | 25 mg |
| Liquid sugar | 150 g |
| Citric acid | 3 g |
| Flavor | 1 g |

Water is added to make a volume of 1000 ml.

### Industrial Applicability

The present invention provides antidepressants and foods and drinks for antidepression.

## Claims

1. An antidepressant comprising, as an active ingredient, a compound represented by formula (I): 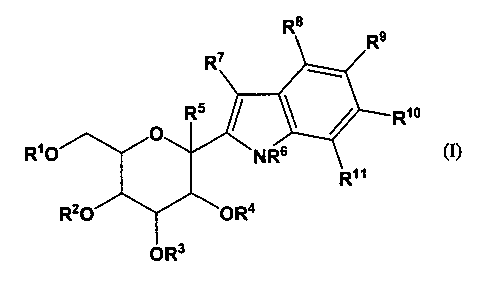 (wherein R¹, R², R³ and R⁴, which may be the same or different, each represent a hydrogen atom, substituted or unsubstituted lower alkyl, substituted or unsubstituted alkenyl, or substituted or unsubstituted aryl; R⁵ represents a hydrogen atom, hydroxy, or substituted or unsubstituted lower alkoxy; R⁶ represents a hydrogen atom, substituted or unsubstituted lower alkyl, substituted or unsubstituted sulfamoyl, substituted or unsubstituted arylsulfonyl, substituted or unsubstituted lower alkoxycarbonyl, or substituted or unsubstituted aralkyloxycarbonyl; R⁷ represents a hydrogen atom or substituted or unsubstituted lower alkyl; and R⁸, R⁹, R¹⁰ and R¹¹, which may be the same or different, each represent a hydrogen atom or substituted or unsubstituted lower alkyl) or a salt thereof.

2. A food and drink for antidepression comprising, as an active ingredient, a compound represented by formula (I) : 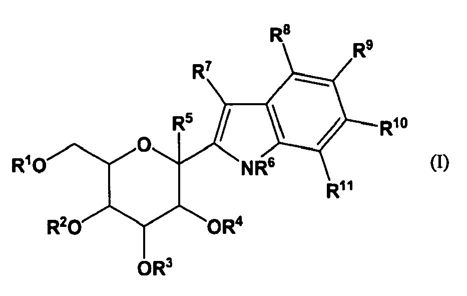 (wherein R¹, R², R³, R⁴, R⁵, R⁶, R⁷, R⁸, R⁹, R¹⁰ and R¹¹ each have the same meaning as defined above) or a salt thereof.

3. The antidepressant according to Claim 1, wherein R⁵ is a hydrogen atom or hydroxy; and R⁸, R⁹, R¹⁰ and R¹¹ each are a hydrogen atom.

4. The antidepressant according to Claim 1, wherein R⁷, R⁸, R⁹, R¹⁰ and R¹¹ each are a hydrogen atom.

5. The food and drink for antidepression according to Claim 2, wherein R⁵ is a hydrogen atom or hydroxy; and R⁸, R⁹, R¹⁰ and R¹¹ each are a hydrogen atom.

6. The food and drink for antidepression according to Claim 2, wherein R⁷, R⁸, R⁹, R¹⁰ and R¹¹ each are a hydrogen atom.

7. An antidepressant comprising, as an active ingredient, a compound represented by formula (Ia) or (Ib) : 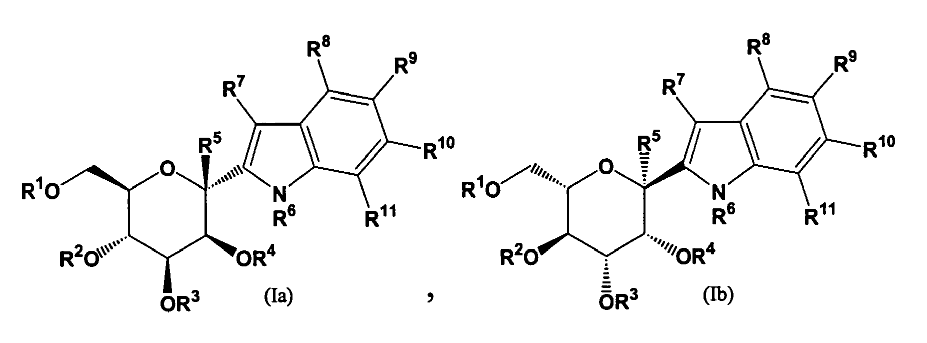 (wherein R¹, R², R³, R⁴, R⁵, R⁶, R⁷, R⁸, R⁹, R¹⁰ and R¹¹ each have the same meaning as defined above) or a salt thereof.

8. A food and drink for antidepression comprising, as an active ingredient, a compound represented by formula (Ia) or (Ib): 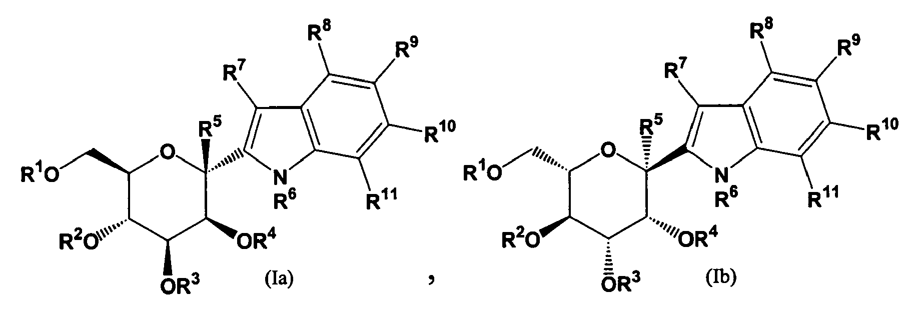 (wherein R¹, R², R³, R⁴, R⁵, R⁶, R⁷, R⁸, R⁹, R¹⁰ and R¹¹ each have the same meaning as defined above) or a salt thereof.

9. An antidepressant comprising, as an active ingredient, 2-amino-3-[2-(α-D-mannopyranosyl)indol-3-yl]propionic acid represented by formula (II): 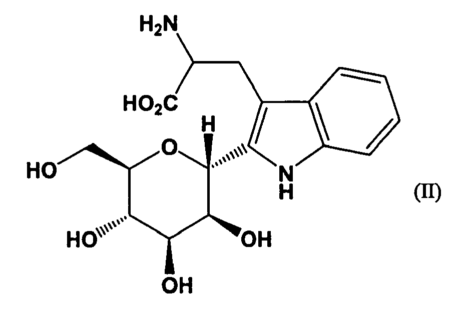 or a salt thereof.

10. An antidepressant comprising, as an active ingredient, 2-amino-3-[2-(a-L-mannopyranosyl)indol-3-yl]propionic acid represented by formula (III): 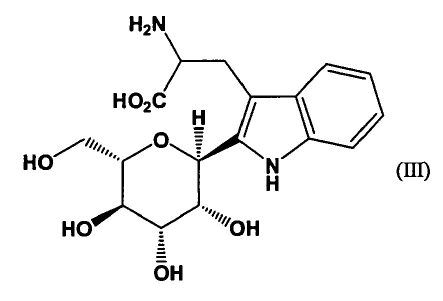 or a salt thereof.

11. A food and drink for antidepression comprising, as an active ingredient, 2-amino-3-[2-(α-D-mannopyranosyl)indol-3-yl]propionic acid represented by formula (II): 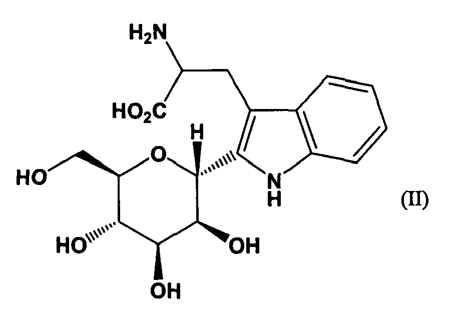 or a salt thereof.

12. A food and drink for antidepression comprising, as an active ingredient, 2-amino-3-[2-(*α*-L-mannopyranosyl)indol-3-yl]propionic acid represented by formula (III): 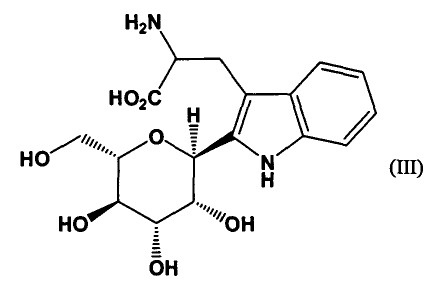 or a salt thereof.

13. An antidepressant comprising, as an active ingredient, a compound represented by formula (IV): 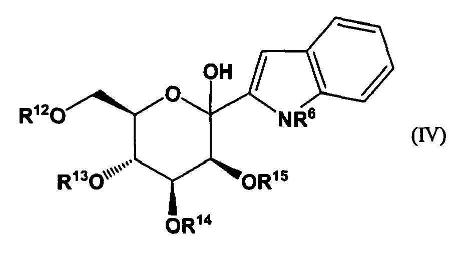 (wherein R⁶ has the same meaning as defined above; and R¹², R¹³, R¹⁴ and R¹⁵, which may be the same or different, each represent substituted alkenyl, or substituted or unsubstituted aryl) or a salt thereof.

14. An antidepressant comprising, as an active ingredient, a compound represented by formula (V): 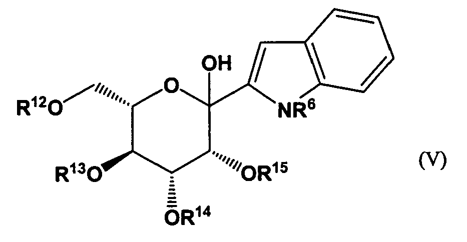 (wherein R⁶, R¹², R¹³, R¹⁴ and R¹⁵ each have the same meaning as defined above) or a salt thereof.

15. A food and drink for antidepression comprising, as an active ingredient, a compound represented by formula (IV) : 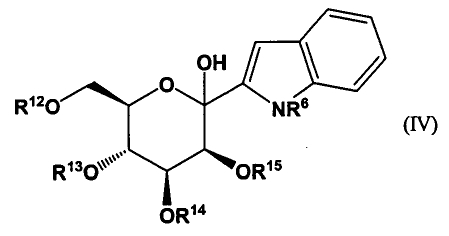 (wherein R⁶, R¹², R¹³, R¹⁴ and R¹⁵ each have the same meaning as defined above) or a salt thereof.

16. A food and drink for antidepression comprising, as an active ingredient, a compound represented by formula (V) : 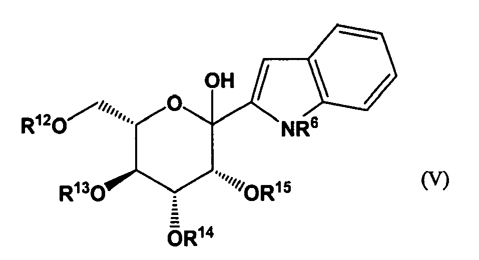 (wherein R⁶, R¹², R¹³, R¹⁴ and R¹⁵ each have the same meaning as defined above) or a salt thereof.

17. A method of preventing or treating depression which comprises administering an effective amount of a compound represented by formula (I) according to Claim 1, formula (Ia) or (Ib) according to Claim 7, formula (II) according to Claim 9, formula (III) according to Claim 10, formula (IV) according to Claim 13 or formula (V) according to Claim 14 or a salt thereof.

18. Use of a compound represented by formula (I), (Ia), (Ib), (II), (III), (IV) or (V) or a salt thereof for the manufacture of the antidepressant according to any of Claims 1, 3, 4, 7, 9, 10, 13 and 14.

19. Use of a compound represented by formula (I), (Ia), (Ib), (II), (III), (IV) or (V) or a salt thereof for the manufacture of the food and drink for antidepression according to any of Claims 2, 5, 6, 8, 11, 12, 15 and 16.
